# EUROPEAN PATENT APPLICATION

(11) **EP 1 923 074 A1**
(43) Date of publication of application: **21.05.2008**
(21) Application number: 06782462.3
(22) Date of filing: 08.08.2006
(51) Int. Cl.: A61K 47/12, A61K 9/20, A61K 47/02, A61K 47/38, A61K 31/166, A61K 31/4152, A61K 31/4196, A61K 31/546

(54) **ORALLY DISINTEGRATABLE TABLET**

(30) Priority: 10.08.2005 JP 2005232083; 21.12.2005 JP 2005367963
(71) Applicant: SHIONOGI & CO., LTD., Osaka-shi, Osaka 541-0045 (JP)
(72) Inventor: IMAMOTO, Chieko, Amagasaki-shi, Hyogo 6600813 (JP); TOYODA, Toshitada, Amagasaki-shi, Hyogo 6600813 (JP); TOMODA, Yoshitaka, Settsu-shi, Osaka 5660022 (JP)
(74) Representative: HOFFMANN EITLE
(86) International application number: PCT/JP2006/315620
(87) International publication number: WO 2007/018192

(57) **Abstract**

By mixing an active ingredient, crystalline cellulose, an inorganic excipient, carmellose, and a lubricant at not more than 0.8% by weight per tablet, and compressing the mixture, or compressing the mixture by an external lubricating method, a palatable orally disintegrating tablet which maintains a tablet hardness and exhibits good disintegrating property can be obtained.

## Description

### Technical Field

Since an elderly or an infant has the low swallowing ability, ingestion of a tablet is difficult. A preparation which can be easily ingested by such the elderly or infant, is rapidly disintegrated in an oral cavity also in an adult having the swallowing ability, and can be ingested almost without feeling bitter taste is desired, and some preparations have been already known.

### Background Art

Upon preparation of an orally disintegrating tablet, in order to guarantee the disintegrating property, sugars and/or a disintegrating agent is generally used. As publication disclosing an orally disintegrating tablet containing sugars or a disintegrating agent, for example, there are following publications.
Patent Publication 1 describes an orally disintegrating tablet containing a pharmaceutical component, erythritol, crystalline cellulose and a disintegrating agent.
Patent Publication 2 describes an orally disintegrating tablet containing a pharmaceutical component, D-mannitol, celluloses and a disintegrating agent. The present Publication is publication disclosing that D-mannitol having an average particle diameter of 30 to 300 µm is preferable.
Patent Publication 3 describes a process for producing an orally disintegrating tablet containing a granule obtained by spraying sugars having high moldability as a binder to sugars having low moldability.
Patent Publication 4 describes that an orally disintegrating tablet can be obtained by compressing a powder obtained by spray-drying a suspension in which an inorganic substance and sugars are uniformly dispersed, together with crystalline cellulose and disintegrating agent. On the other hand, it is described that a tablet obtained by directly compressing a simple mixture consisting the same composition has a deteriorated hardness.
Patent Publication 5 describes that an orally disintegrating tablet can be obtained by mixing a surface modifying base such as light anhydrous silicic acid to a drug efficacy component, modifying a surface using a high speed stirring granulator or the like, adding a disintegrating agent to the thus obtained surface-modified powder, and directly compressing this. It is described that, as a disintegrating agent, partially pregelatinized starch and crospovidone are most suitable.
Patent Publication 6 describes that a tablet having good disintegrating property can be obtained by blending a disintegrating agent into a granule containing a water-easily soluble drug, further adding a cellulose powder and/or an inorganic additive, and compressing this.
In addition, Non-Patent Publication 1 (Pamphlet of Kyowa Chemical Industry Co.,Ltd. (excipient for direct compression, anhydrous dibasic calcium phosphate GS)) describes data of a preparation in which crystalline cellulose, anhydrous dibasic calcium phosphate, carmellose, and 1% by weight of magnesium stearate are blended.

Patent Publication 1 :
   Japanese Patent Application Laid-Open (JP-A) No.10-182436
Patent Publication 2 :
   JP-A No.2001-58944
Patent Publication 3 :
   WO 95/20380
Patent Publication 4 :
   JP-A No.2000-86537
Patent Publication 5 :
   WO 00/54752
Patent Publication 6 :
   JP-A No.2002-12540
Non-Patent Publication 1 :
   Pamphlet of Kyowa Chemical Industry Co.,Ltd. (excipient for direct compression, anhydrous dibasic calcium phosphate GS)

Patent Publications 1 and 2 are a formulation example in which sugars which is an additive most frequently used in formulation of an orally disintegrating tablet and a disintegrating agent are blended, and a disintegration rate is increased.
In preparations of Patent Publications 3, 4 and 5, an additional preparation step is required in order to improve the disintegrating property and hardness of a tablet, and productivity is deteriorated.
In addition, in Patent Publication 6, a disintegration time of a preparation greatly exceeds three minutes and a disintegration time as an intraoral disintegration time is not satisfied.
Non-Patent Publication 1 describes a tablet in which anhydrous dibasic calcium phosphate, crystalline cellulose, carmellose, and magnesium stearate which is a lubricant are blended as described above, but a blending amount of magnesium stearate is 1% and there is a possibility that the disintegrating property of a tablet is reduced under warming and humidification.

### Disclosure of Invention

### Problems to be solved by the Invention

An object of the present invention is to provide an orally disintegrating tablet which has a suitable hardness, rapid disintegration in an oral cavity and good ingestion feeling without blending a soluble additive such as sugars, and can be produced by the existing facility without necessity of a special preparation machine.

### Means to solve the Problems

It was found out that, by directly compression-molding a powder comprising an active ingredient, crystalline cellulose, an inorganic excipient, particularly carmellose as a disintegrating agent, a lubricant at not more than 0.8% by weight, preferably not more than 0.5% by weight, more preferably not more than 0.1% by weight per tablet, an orally disintegrating tablet exhibiting good disintegrating property and maintaining a tablet hardness can be obtained. In the above case, by adopting a blending amount of anhydrous dibasic calcium phosphate as an inorganic excipient, and a stearic acid metal salt as a lubricant of not more than 0.8% by weight, preferably not more than 0.5% by weight, more preferably not more than 0.1% by weight per tablet, and particularly, by blending an active ingredient, crystalline cellulose, anhydrous dibasic calcium phosphate, carmellose, and magnesium stearate at not more than 0.1% by weight per tablet, and compression-molding the blend by an external lubricating method, an orally disintegrating tablet exhibiting good disintegrating property and maintaining a tablet hardness could be obtained.

That is, the present invention related to:
(1) a tablet characterized in that an active ingredient, crystalline cellulose, inorganic excipient, carmellose and a lubricant at not more than 0.8% by weight per tablet are contained,
(2) the tablet according to (I), wherein the inorganic excipient is anhydrous dibasic calcium phosphate,
(3) the tablet according to (1) or (2), wherein a lubricant at not more than 0.5% by weight per tablet is blended,
(4) the tablet according to (3), wherein a lubricant at not more than 0.1% by weight per tablet is blended,
(5) the tablet according to any one of (1) to (4), wherein an average particle diameter of crystalline cellulose is 10 to 150 µm,
(6) the tablet according to any one of (2) to (5), wherein a bulk density of anhydrous dibasic calcium phosphate is 0.3 to 1.0 g/mL,
(7) the tablet according to any one of (1) to (6), wherein carmellose is blended at 1 to 30% by weight per tablet,
(8) the tablet according to any one of (1) to (7), wherein the lubricant is a stearic acid metal salt,
(9) the tablet according to (8), wherein the stearic acid metal salt is magnesium stearate,
(10) the tablet according to any one of (1) to (9), wherein a sweetener is blended,
(11) the tablet according to (10), wherein the sweetener has a sweetness degree which is 50-fold more, letting a sweetness of white sugar to be 1,
(12) the tablet according to (11), wherein the sweetener is acesulfame potassium or sucralose,
(13) the tablet according to any one of (10) to (12), wherein a blending amount of the sweetener is not more than 10% by weight per tablet,
(14) the tablet according to any one of (1) to (13), wherein a method of adding a lubricant is an external lubricating method,
(15) the tablet according to (14), wherein an active ingredient, crystalline cellulose, anhydrous dibasic calcium phosphate, carmellose, and magnesium stearate at not more than 0.1% by weight per tablet are contained.
(16) the tablet according to any one of (1) to (15), which is an orally disintegrating tablet,

### Effect of the Invention

The tablet of present invention can be easily ingested without water, is rapidly disintegrated in an oral cavity, has a suitable hardness and has good ingestion feeling. For this reason, the tablet can be used as an orally disintegrating tablet. Moreover, the tablet has a simple process for producing tablet.

The "orally disintegrating tablet containing an active ingredient, crystalline cellulose, an inorganic excipient, carmellose, and a lubricant at not more than 0.8% by weight per tablet" means a formulation which exerts the effect of the present invention (maintenance of good disintegrating property of a tablet, and a suitable tablet hardness) by inclusion of an active ingredient, crystalline cellulose, an inorganic excipient, carmellose, and a lubricant at not more than 0.8% by weight pre tablet. An essential feature is an active ingredient, crystalline cellulose, an inorganic excipient, carmellose, and a lubricant at not more than 0.8% by weight per tablet, and other additive may be contained in such a range that the effect of the present invention is not influenced.

Examples of crystalline cellulose used in the tablet of the present invention include Ceolus PH101, Ceolus PH102, Ceolus PH301, Ceolus PH302, Avicel PHF20, JP, Ceolus KG802 (manufactured by Asahi Kasei Corporation), VIVAPUR (Grade 105, 101, 103, 301, 102, 112), ARBOCEL (Grade M80, P290, A300), Prosolv SMCC50, Prosolv SMCC90 (manufactured by JRS PHARMA) and the like. These crystalline celluloses may be used alone, or two or more kinds may be used jointly. An average particle diameter of crystalline cellulose before tablet production is preferably 10 to 150 µm, more preferably 30 to 130 µm, particularly preferably 40 to 120 µm. When a diameter is greater or smaller than this average particle diameter, there is a possibility that hardness of a tablet is reduced, and a disintegration time is delayed. Specifically, Ceolus PH102 (manufactured by Asahi Kasei Corporation, average particle diameter about 100 µm) is preferable.

Examples of the inorganic excipient used in the tablet of the present invention include anhydrous dibasic calcium phosphate, magnesium aluminate metasilicate, synthetic hydrotalcite, precipitated calcium carbonate, and magnesium carbonate, particularly preferably anhydrous dibasic calcium phosphate, such as anhydrous dibasic calcium phosphate GS (manufactured by Kyowa Chemical Industry Co., Ltd.) Fujicalin (manufactured by Fuji Chemical Industry Co., Ltd.), anhydrous dibasic calcium phosphate light (manufactured by Kyowa Chemical Industry Co., Ltd.), anhydrous dibasic calcium phosphate heavy (manufactured by Kyowa Chemical Industry Co., Ltd.) and the like. These inorganic excipients may be used alone, or two or more kinds may be used jointly. A bulk density of the inorganic excipient before tablet production is preferably 0.30 to 1.0 g/mL, more preferably 0.5 to 1.0 g/ml, particularly preferably 0.6 to 1.0 g/mL. When a bulk density is lower or higher than this bulk density, there is a possibility that hardness of a tablet is reduced, and a disintegration time is delayed. Specifically, anhydrous dibasic calcium phosphate GS (manufactured by Kyowa Chemical Industry Co., Ltd., bulk density 0.71 to 1.0 g/mL) is preferable.

In the tablet of the present invention, a content of crystalline cellulose and anhydrous dibasic calcium phosphate which is an inorganic excipient can be easily determined. For example, predetermined amounts of crystalline cellulose, anhydrous dibasic calcium phosphate and carmellose are appropriately mixed with an active ingredient, the mixture is compression-molded, and hardness and the disintegrating property are conformed, thereby, suitability thereof can be easily determined.
Since a content of crystalline cellulose and an inorganic excipient also depends on a physical nature of an active ingredient, it is preferable to appropriately determine content as described above. Particularly, it is preferable to use crystalline cellulose and an inorganic excipient at 30 to 99.9% by weight based on a total weight of the tablet. Particularly, it is preferable to use crystalline cellulose and an inorganic excipient at 50 to 99.9% by weight. At these contents, a physical nature of an active ingredient hardly influences thereon, and the present preparation exhibits particularly good disintegration rate and tablet hardness.
In addition, it is preferable to appropriately determine a ratio of blending crystalline cellulose and anhydrous dibasic calcium phosphate as described above. It is enough that a ratio by weight of crystalline cellulose and anhydrous dibasic calcium phosphate is in a range of 8:2 to 2:8. In the above range, an orally disintegrating tablet having good disintegration rate and tablet hardness can be obtained. When a ratio of blending crystalline cellulose is higher than this, there is a possibility that mouth feeling is worsened due to roughness of crystalline cellulose and, when a ratio of blending crystalline cellulose is lower than this, there is a possibility that a tablet hardness is reduced. Preferable is a tablet in which crystalline cellulose and anhydrous dibasic calcium phosphate are blended at a ratio by weight of 5:5 to 3:7, and more preferable is a tablet in which crystalline cellulose and anhydrous dibasic calcium phosphate are blended at a ratio by weight of about 4:6.
A weight of an active ingredient may be any amount, and is 0.1 to 50% by weight, preferably 0.1 to 40% by weight, more preferably 0.1 to 30% by weight relative to a total weight of a tablet. In this case, the tablet hardly undergoes influence by a physical nature of an active ingredient, and the present preparation exhibits particularly good disintegration rate and tablet hardness.

In the tablet of the present invention, a disintegrating agent to be used, carmellose is preferable. Carmellose has another name of carboxymethylcellolose. Carmellose may be according to Japanese Pharmacopoeia 14^{th} revision. Specifically, carmellose is NS-300 (Gotoku Chemical Company LTD.).

A content of carmellose is 1 to 30% by weight, preferably 5 to 25% by weight, more preferably 7.5 to 20% by weight per tablet. When an amount is smaller than this blending amount, there is possibility that a disintegration time of the tablet becomes longer and, when an amount is larger than this content, there is a possibility that hardness of the tablet is reduced.

As a lubricant to be used in the tablet of the present invention, there are sucrose esters of fatty acid ester, talc, hydrated silicon dioxide, stearic acid metal salt and the like, preferably stearic acid metal salt. Examples of the stearic acid metal salt include magnesium stearate, calcium stearate and the like. Preferable is magnesium stearate.

A content of the stearic acid metal salt is not more than 0.8% by weight, preferably not more than 0.5% by weight, more preferably not more than 0.1% by weight per tablet. Specifically, the content is 0.001 to 0.8% by weight, preferably 0.001 to 0.5% by weight, more preferably 0.001 to 0.1% by weight. When a content is larger than this content, there is a possibility that a disintegration time of the tablet becomes longer.

As the active ingredient to be used in the orally disintegrating tablet of the present invention, any active ingredient can be used. The active ingredient is not particularly limited as far as it is an active ingredient which can be administered orally. Examples include an antibiotic, a chemotherapeutic, a hypnotic-sedative, an anti-psyshosis agent, an anti-anxiety agent, an antiepileptic, an antipyretic-analgesic-antiphlogistic, an anti-Parkinson agent, a mental and nervous agent, a skeletal muscle relaxant, an autonomic agent, an antispasmodic, a cardiotonic agent, an arrhythmia agent, a diuretic, an antihypertensive, a vessel reinforcing agent, a vasoconstrictor, a vasodilator, a hyperlipemia agent, an antitussive-expectorant, a bronchodilator, a stegnotic, a medicine for intestinal disorders, a digestive ulcer agent, a stomachic digestion agent, an antacid, a cholagogue, a medicine for digestive system, a vitamin agent, a nutritional drug, a hepatic disease agent, a gout treating agent, a diabetes agent, a tumor drug, an anti-histamine agent, a crude drug, and an osteoporosis agent.

The tablet of the present invention may further contain various additives which are generally used in production of the tablet, if necessary. For example, the tablet may contain an additive at 0.1 to 30% by weight (preferably 0.1 to 10% by weight, particularly preferably 0.1 to 5.0% by weight) based on a total weight of the tablet. In addition, these substances may be used alone, or by mixing them at an arbitrary ratio. Examples of the additive include a sweetener, a corrigent, a perfume, a lubricant, a binder, a flowing agent, a coloring agent, and a coating agent.
The sweetener means a glucide including sugars and sugar alcohols, and other non-glucide. Since the present preparation does not contain sugars and sugar alcohols as an excipient, it is difficult to generate sufficient sweetness using sugars and sugar alcohol. For this reason, in the tablet of the present invention, particularly in an orally disintegrating tablet, a substance by which strong sweetness is felt at a smaller amount as compared with sugars and sugar alcohols is preferable, and a non-glucide natural sweetener and synthetic sweetener are preferable. Specifically, the substance is a sweetener having a sweetness degree which is 50-fold more, letting a sweetness degree of white sugar to be 1. Examples include acesulfame potassium, aspartame, saccharin or salt thereof, glycyrrhizic acid or a salt thereof, stevia or a salt thereof, sucralose and thaumatin. A content of the sweetener is not more than 10% by weight, preferably 0.1 to 10% by weight, more preferably 0.5 to 7.5% by weight per tablet.
Examples of the corrigent include ascorbic acid and a salt thereof, glycine, sodium chloride, magnesium chloride, hydrochloric acid, diluted hydrochloric acid, citric acid and a salt thereof, anhydrous citric acid, L-glutamic acid and a salt thereof, succinic acid and a salt thereof, acetic acid, tartaric acid and a salt thereof, sodium hydrogen carbonate, fumaric acid and a salt thereof, malic acid and a salt thereof, glacial acetic acid, disodium inosinate, and honey.
The perfume includes a so-called flavoring agent, and examples include orange essence, orange oil, caramel, camphor, cinnamon oil, spearmint oil, strawberry essence, chocolate essence, cherry flavor, spruce oil, pine oil, mint oil, vanilla flavor, bitter essence, fruit flavor, peppermint essence, mix flavor, mint flavor, menthol, lemon powder, lemon oil, and rose oil.
Examples of the binder include gum arabic, gum arabic powder, partially gelatinized starch, gelatin, agar, dextrin, pullulan, povidone, polyvinyl alcohol, ethylcellulose, carboxymethylcellulose, carmellose, carmellose sodium, hydroxyethylcellulose, hydroxyethylmethylcellulose, hydroxypropylcellulose and hydroxypropylmethylcellulose.
Examples of the flowing agent include hydrated silicon dioxide, light anhydrous silicic acid, heavy anhydrous silicic acid, and titanium oxide.
Examples of the coloring agent include edible coloring agents such as food Red No.3, food Yellow No.5, and food Blue No.1, yellow ferric oxide, red ferric oxide, brown iron oxide, black iron oxide, copper chlorophyll, copper cholophyllin sodium, riboflavine, and powdered tea.
Examples of the coating agent include polyvinyl alcohol, ethylcellulose, carboxymethylethylcellulose, carmellose, carmellose sodium, hydroxyethylcellulose, hydroxyethylmethylcellulose, hydroxypropylcellulose and hydroxypropylmethylcellulose, PVA-copolymer, ethyl acrylate methyl methacrylate copolymer dispersion, aminoalkyl methacrylate copolymer, opadry, carnauba wax, carboxyvinyl polymer, dry methacrylic acid copolymer, dimethylaminoethyl methacrylate methyl methacrylate copolymer, stearyl alcohol, shellac, cetanol, hydroxypropylmethylcellulose acetate succinate, hydroxypropylmethylcellulose phthalate, fumaric acid·stearic acid polyvinyl acetal diethyl aminoacetate hydroxypropylmethylcellulose mixture, polyvinyl acetal diethyl aminoacetate, polyvinyl alcohol, methacrylic acid copolymer, and 2-methyl-5-vinylpylidine methyl acrylate methacrylic acid copolymer.

These components can be usually used alone, or by mixing them at an arbitrary amount in such a range that the disintegrating property and moldability in the tablet of the present invention are not deteriorated. Preferable are combinations of crystalline cellulose/anhydrous dibasic calcium phosphate/carmellose/magnesium stearate/ acesulfame potassium, crystalline cellulose /anhydrous dibasic calcium phosphate/carmellose/magnesium stearate/ acesulfame potassium/mint oil/hydrated silicon dioxide, crystalline cellulose/anhydrous dibasic calcium phosphate/carmellose/magnesium stearate/sucralose, crystalline cellulose/anhydrous dibasic calcium phosphate/carmellose/magnesium stearate/sucralose/mint oil/hydrated silicon dioxide. When additives of these combinations are used, it is possible to produce an orally disintegrating tablet which has a great disintegration rate, has a high hardness, and can mask bitter taste.

In an orally disintegrating tablet, generally, sugars, and sugar alcohols is used as an excipient. On the other hand, the orally disintegrating tablet of the present invention is an orally disintegrating tablet substantially consisting of an active ingredient, crystalline cellulose and an inorganic excipient. That is, the present invention features that sugars as an excipient which is generally used in an orally disintegrating tablet is not contained. In the orally disintegrating tablet of the present invention, sugars such as sucrose, glucose, fructose, thick malt syrup, lactose and the like is not used as an excipient.
In addition, the orally disintegrating tablet of the present invention features that it does not contain sugar alcohols as an excipient which is generally used in an orally disintegrating tablet. In the orally disintegrating tablet of the present invention, sugar alcohols such as erythritol, D-sorbitol, xylitol, D-mannitol, maltitol and the like is not used as an excipient.

### Best Mode for Carrying Out the Invention

A process for producing the orally disintegrating tablet of the present invention will be described below.
Examples of a specific production process include a process for producing the tablet, by weighing an active ingredient and a preparation raw material, mixing them with a suitable mixer such as a V-type mixer, and directly compressing a thus-obtained mixed powder for tablet using a compressing machine described later. In addition, in order to obtain a mixed powder for tablet, a method of vigorously mixing a raw material with a stirring granulator and a method of mixing and grinding a material with a grinder, a method of compression granulation with a dry granulator, a method of performing wet granulation using water, acetone, ethyl alcohol, propyl alcohol, or a mixture thereof in which a binder is dispersed or dissolved if necessary, and a method of producing a mixed powder for tablet by classifying two or more groups may be used. When a mixed powder for tablet is produced, if necessary, a binder, a corrigent, a flowing agent, a lubricant, a perfume, a sweetener and a coloring agent may be mixed.
In addition, regarding compression molding, according to formulation of the present invention, even when a lubricant is a small amount (not more than 0.8% by weight, preferably not more than 0.5% by weight, more preferably not more than 0.1% by weight), a conventional compressing method (an internal mixing method) and an external lubricating method of adhering a lubricant to a mortal and a mallet of a compressing machine can be used. As an apparatus for performing an external lubricating method, there is ELSP1-type III manufactured by KIKUSUI SEISAKUSHO LTD.
When an addition amount of a lubricant is reduced, a disintegration rate can be further increased, and a tablet hardness can be improved and, furthermore, stability of a drug can be enhanced. In the conventional procedure or formulation of mixing a mixed powder for tablet with a lubricant, a lubricant at 1 to 3 mg relative to 100 mg of the tablet is required, but in the present formulation, compression is possible with a lubricant at a small amount of not more than 0.8% by weight, preferably not more than 0.5% by weight, more preferably not more than 0.1% by weight per tablet. Particularly, it is further preferable that active ingredient, crystalline cellulose, anhydrous dibasic calcium phosphate, carmellose, and magnesium stearate at not more than 0.1% by weight per tablet are blended, and a method of adding a lubricant is an external lubricating method.

Among the foregoing, particularly, it is preferable to mold a powder substantially containing an active ingredient, crystalline cellulose, anhydrous dibasic calcium phosphate, and carmellose (in the case of addition of a sweetener, acesulfame potassium or sucralose; in the case of addition of a perfume, a mint oil) by an internal mixing method or an external lubricating method. In the present invention, a particle diameter of an active ingredient and an additive is not particularly limited.

The thus obtained mixed powder for tablet is compression-molded at a compression pressure of 200 kg to 1500 kg using, for example, an apparatus for performing external lubricating compression, a single compressing machine, or a rotary compressing machine. When a pressure is lower than this, a tablet hardness is deficient, and a sufficient hardness for handling can not be maintained and, when a pressure is high, disintegration is delayed, being not preferable.
Regarding molding of the orally disintegrating tablet of the present invention, any shape can be adopted; for example, a laminated tablet or dry-coated tablets having a shape of a circle, an ellipse, a sphere, a bar or a donut may be used, and further, the tablet may be covered by coating. In addition, impression such as a mark and a letter for improving discriminability, or a cleavage line for revision may be imparted.

In the present invention, a dosage form (e.g. powder particle) containing an active ingredient in which bitter taste is masked is produced in advance, thereafter, the aforementioned component together with a preparation may be mixed to produce a tablet. In this case, it is possible to produce an orally disintegrating tablet in which bitter taste is masked.

The tablet of the present invention is useful as an orally disintegrating tablet, is rapidly disintegrated with saliva in an oral cavity, and can be smoothly ingested without leaving rough feeling in the mouth. Dissolution in mouth of the orally disintegrating tablet of the present invention is usually 1 to 60 seconds, preferably 1 to 40 seconds, further preferably around 1 to 30 seconds.
In addition, it is known that hardness (measured value with tablet hardness tester) usually has no problem when the hardness is a value of around 30 to 70 N, but the orally disintegrating tablet of the present invention has hardness of around 10 to 200 N, more preferably 30 to 150 N.
This preparation can be ingested without disintegration in an oral cavity, and can be ingested with water.

### Examples

The present invention will be explained in more detail below by way of Examples and Comparative Examples, but these do not limit the present invention.
Tablets obtained in Examples and Comparative Examples were tested for a tablet hardness, a disintegrating time and organoleptic property by the following testing methods.
(1) Hardness test
   The hardness was measured using hardness measuring exclusive machine (manufactured by ERWEKA International AG). A test was performed using 10 tablets, and an average was shown (standard; not less than 30 N).
(2) Disintegration test
   According to a disintegration test method described in Japanese Pharmacopoeia l4^{th} revision, a disintegration time of six tablets was measured and maximum was shown (standard; within 30 seconds)
(3) Introral disintegrating test
   Six healthy adults held the tablet in mouth, and a disintegration time of the tablet in an oral cavity was measured. Table shows a maximum of disintegration time (standard; within 30 seconds).
(4) Organoleptic test
   Six healthy adults hold the orally disintegrating tablet in mouth, dry feeling in an oral cavity at ingestion are classified according the following assessment criteria, and most frequent assessment is selected. In addition, the tablet held in an oral cavity is discharged after a test, and an oral cavity is washed with water.
   ○: Dryness is not feld.
   Δ: Dryness is slightly feld.
   ×: Uncomfortable feeling is felt.

Using formulation Table 1 as fundamental formulation, in order to study (i) a kind of a disintegrating agent, (ii) an average particle diameter of crystalline cellulose, (iii) a bulk density of anhydrous dibasic calcium phosphate and (iv) a kind of a sweetener, tablets were variously produced, and hardness test, a disintegration test and an intraoral disintegration test were performed.

### (Fundamental formulation)

**[Table 1]**

| Component | Blending amount |
|---|---|
| Ethenzamide | 10 |
| (I) Disintegrating agent | 10 |
| (II) Crystalline cellulose | 31 |
| (III) Anhydrous dibasic calcium phosphate | 47.6 |
| (IV) Sweetener | 1.4 |
| Magnesium stearate | Minor amount (0.1) |
| Total amount (mg) | 100 |

### (Tablet production process)

Ethenzamide, a disintegrating agent, crystalline cellulose, anhydrous dibasic calcium phosphate, and a sweetener were weighed according to a blending amount described in Table 1, and mixed in a polyethylene bag to produce a powder for tablet. Then, using an experimental small compressing machine VELA5 (manufactured by KIKUSUI SEISAKUSHO LTD.) equipped with an external lubricating apparatus ELSP1-type III (manufactured by KIKUSUI SEISAKUSHO LTD.), tablets each weighing 100 mg were produced under the condition that not more than 0.1 mg magnesium stearate is adhered per tablet. Thereupon, a shape of a mallet was round, and a diameter was 6.5 mm.

### (Study of disintegrating agent)

### (Example 1, Comparative Examples 1-5)

In Example 1, and Comparative Examples 1-5, as shown in Table 2, powders for tablet in which a kind of disintegrating agent was changed were compressed, and hardness, a disintegration time, an intraoral disintegration time and dry feeling in an oral cavity of the tablets were assessed. As crystalline cellulose, Ceolus PH102 (manufactured by Asahi Kasei Corporation) was used. As anhydrous dibasic calcium phosphate, anhydrous dibasic calcium phosphate GS (manufactured by Kyowa Chemical Industry Co., Ltd.) was used. As a sweetener, acesulfame potassium (manufactured by Nutrinova Japan Ltd.) was used. The powder was compressed at a compression pressure of 5 to 7 kN.

### (Experimental result)

Experimental results are shown in Table 2. From this result, when carmellose was used, hardness of the tablet was high, a disintegration time and an intraoral disintegration time were short, and there was no dry feeling in an oral cavity.

**[Table 2]**

| | Example 1 | Comparative Example 1 | Comparative Example 2 | Comparative Example 3 | Comparative Example 4 | Comparative Example 5 |
|---|---|---|---|---|---|---|
| Disintegrating agent name | Carmellose | Carmellose calcium | Croscarmellose sodium | Low·substituted hydroxyporopylc ellulose | Carboxymethyl starch sodium | Crospovidone |
| Hardness (N) | 52.0 | 52.5 | 54.0 | 53.0 | 46.8 | 67.3 |
| Disintegration time (sec) | 5 | 16 | 19 | 14 | 22 | 13 |
| Intraoral disintegration time (sec) | 6 | 15 | 14 | 12 | 18 | 7 |
| Dry feeling | ○ | Δ | × | Δ | Δ | × |

| | | | | | | |
|---|---|---|---|---|---|---|
| **·** Carmellose: NS-300 (manufactured by Gotoku Chemical Company LTD.) **·** Carmellose Calcium: ECG505 (manufactured by Gotoku Chemical Company LTD.) · Croscarmellose sodium: Ac-Di-Sol (manufactured by Asahi Kasei Corporation) · Low-substituted hydroxypropylcellulose: LH11 (manufactured by Shin-Etsu Chemical Co., Ltd.) **·** Carboxystarch sodium: EXPLOTAB (manufactured by Kimura Sangyo Co., Ltd.) - Crospovidone: Polyplasdone XL (manufactured by ISP) | | | | | | |

### (Study of average particle diameter of crystalline cellulose)

### (Examples 1-3)

In Examples 1 to 3, as shown in Table 2, powder components of tablets in which an average particle diameter of crystalline cellulose was changed were compressed, and hardness, a disintegration time and an intraoral disintegration time of the tablets were measured. As carmellose, NS-300 (manufactured by Gotoku Chemical Company LTD.) was used. As anhydrous dibasic calcium phosphate, anhydrous dibasic calcium phosphate GS (manufactured by Kyowa Chemical Industry Co., Ltd.) was used. As a sweetener, acesulfame potassium manufactured by Nutrinova Japan Ltd.) was used. A process of producing the tablet is the same as that of Example 1.

### (Experimental result)

Experimental results are shown in Table 3. From this result, hardness, a disintegration time and an intraoral disintegration time satisfied a goal value when any particle diameter crystalline cellulose was used. Particularly, in Ceolus PH-102 having an average particle diameter 100 µm, hardness of the tablet was high, and a disintegration time and an intraoral disintegration time were both short.

**[Table 3]**

| | Example 1 | Example 2 | Example 3 |
|---|---|---|---|
| Average particle diameter (µm) | 100 | 50 | 17 |
| Hardness (N) | 52.0 | 47.0 | 46.5 |
| Disintegration time (sec) | 5 | 14 | 14 |
| Intraoral disintegration time (sec) | 6 | 20 | 25 |

| | | | |
|---|---|---|---|
| · Example 1: Ceolus PH-102 (manufactured by Asahi Kasei Corporation) · Example 2: Ceolus PH-101 (manufactured by Asahi Kasei Corporation) · Example 3: Ceolus F20, JP (manufactured by Asahi Kasei Corporation) | | | |

### (Study of bulk density of anhydrous dibasic calcium phosphate) (Examples 1, 4, 5)

In Examples 1, 4 and 5, as shown in Table 4, powder components of tablets in which an average particle diameter of anhydrous dibasic calcium phosphate was changed were compressed, and hardness, a disintegration time and an intraoral disintegration time of the tablets were measured. As carmellose, NS-300 (manufactured by Gotoku Chemical Company LTD.) was used. As crystalline cellulose, Ceolus PH-102 (manufactured by Asahi Kasei Corporation) was used. As a sweetener, acesulfame potassium (manufactured by Nutrinova Japan Ltd.) was used. In addition, a process for producing the tablet was the same as that of Example 1.

### (Experimental result)

Experimental results are shown in Table 4. From this result, hardness, a disintegration time and an intraoral disintegration time satisfied a goal value when anhydrous dibasic calcium phosphate having an any bulk density was used. Particularly, in anhydrous dibasic calcium phosphate GS having a bulk density of 0.85 g/mL, hardness of the tablet was high, and a disintegration time and an intraoral disintegration time were both short.

**[Table 4]**

| | Example 1 | Example 4 | Example 5 |
|---|---|---|---|
| Bulk density (g/mL) | 0.85 | 0.52 | 0.43 |
| Hardness (N) | 52.0 | 33.3 | 45.0 |
| Disintegration time (sec) | 5 | 17 | 19 |
| Intraoral disintegration time (sec) | 6 | 25 | 29 |

| | | | |
|---|---|---|---|
| · Example 1: anhydrous dibasic calcium phosphate GS (manufactured by Kyowa Chemical Industry Co., Ltd.) · Example 4: anhydrous dibasic calcium phosphate light (manufactured by Kyowa Chemical Industry Co., Ltd.) · Example 5:Fujicalin SG (manufactured by Fuji Chemical Industry Co. Ltd.) | | | |

### (Study of sweetener)

### (Examples 1, 6 to 9, 30)

In Examples 1, 6 to 9, and 30, as shown in Table 5, powder components of tablets in which a kind of a sweetener was changed were compressed, and hardness, a disintegration time and an intraoral disintegration time of the tablets were measured. As carmellose, NS-300 (manufactured by Gotoku Chemical Company LTD.) was used. As crystalline cellulose, Ceolus PH102 (manufactured by Asahi Kasei Corporation) was used. As anhydrous dibasic calcium phosphate, anhydrous dibasic calcium phosphate GS (manufactured by Kyowa Chemical Industry Co., Ltd.) was used. In addition, a process for producing the tablet was the same as that of Example 1.

### (Experimental result)

Experimental results are shown in Table 5. From this result, among these sweeteners, a disintegration time and an intraoral disintegration time of the tablet containing dipotassium glycyrrhizinate of Example 30 were long, and the disintegrating property was deteriorated. Tablets incorporating other sweetener (acesulfame potassium, aspartame, stevia, sucralose, and thaumatin) had a high hardness, and a short disintegration time and intraoral disintegration time.

**[Table 5]**

| | Example 1 | Example 6 | Example 7 | Example 8 | Example 9 | Example 30 |
|---|---|---|---|---|---|---|
| Sweetener | Acesulfame potassium | Aspartame | Thaumatin | Stevia | Sucralose | Dipotassium glycyrrhizinate |
| Hardness (N) | 52.0 | 48.2 | 51.4 | 48.8 | 47.9 | 49.5 |
| Disintegration time (sec) | 5 | 9 | 9 | 10 | 10 | 30 |
| Intraoral disintegration time (sec) | 6 | 10 | 10 | 10 | 10 | 35 |

| | | | | | | |
|---|---|---|---|---|---|---|
| · Example 1: Acesulfame potassium (manufactured by Nutrinova Japan Ltd.) · Example 6: Aspartame (manufactured by Ajinomoto Co.,Inc.) · Example 7: Thaumatin (manufactured by San-Ei Gen F.F.I.,Inc) · Example 8: Stevia (manufactured by MARUZEN PHARMACEUTICALS CO., LTD.) · Example 9: Sucralose (manufactured by San-Ei Gen F.F.I.,Inc) · Example 30: Dipotassium glycyrrhizinate (manufactured by Maruzen Pharmaceutical Co., Ltd.) | | | | | | |

### (Study of process for producing tablet)

Formulation of Examples 1 and 10 is shown in Table 6. As a method of adding magnesium stearate, two methods of an external lubricating method of adhering magnesium stearate to a mallet and a mortar, and rendering magnesium stearate reside only on a surface of the tablet, and an internal mixing method of mixing magnesium stearate and a powder, and rendering magnesium stearate on a whole tablet were performed. Hardness, a disintegration time and an intraoral disintegration time of tablets prepared by these two method were measured.

### (Process for producing tablet)

As a process for producing the tablet of Example 10 in which magnesium stearate is blended in the interior (internally mixed), ethenzamide, carmellose, crystalline cellulose, anhydrous dibasic calcium phosphate, acesulfame potassium, and magnesium stearate were weighed according to a blending amount described in Table 6, and mixed in a polyethylene bag, magnesium stearate was added to mix materials, thereby, a powder for tablet was prepared. Then, tablets each weighing 100 mg were prepared with an experimental small compressing machine VELA5 (manufactured by Kikusui Seisakusho LTD). Thereupon, a mallet having a round shape and a diameter of 6.5 mm was used, and the powder was compressed at a compression pressure of 6 kN.

**[Table 6]**

| | Example 1 | Example 10 |
|---|---|---|
| Method of adding lubricant | External lubricating | Internal mixing |
| Ethenzamide | 10 | 10 |
| Carmellose | 10 | 10 |
| Crystalline cellulose | 31 | 31 |
| Anhydrous dibasic calcium phosphate | 47.6 | 47.6 |
| Acesulfame potassium | 1.4 | 1.4 |
| Magnesium stearate | 0.1 | 0.1 |
| Total amount (mg) | 100.1 | 100.1 |

### (Experimental result)

Experimental results are shown in Table 7. From this result, both of methods of adding magnesium stearate satisfied a goal value of a hardness, a disintegration time and an intraoral disintegration time. Particularly, the tablet produced by an external lubricating method had a high hardness, and a short disintegration time and an intraoral disintegration time.

**[Table 7]**

| | Example 1 | Example 10 |
|---|---|---|
| Method of adding lubricant | External lubricating | Internal mixing |
| Hardness (N) | 52.0 | 50.5 |
| Disintegration time (sec) | 5 | 10 |
| Intraoral disintegration time (sec) | 6 | 9 |

### (Study of addition amount of lubricant)

Formulation of Examples 11 to 14, and Comparative Example 6 is shown in Table 8. Hardness, a disintegration time and an intraoral disintegration time of tablets in which an addition amount of magnesium stearate which is a lubricant was changed to 0.1 mg, 0.3 mg, 0.5 mg, 0.8 mg and 1.0 mg were measured. The method for producing the tablet of Examples 11 to 14 and Comparative Example 7 is same as that in Example 10, and the powder was compressed so that hardness became about 60 N.

**[Table 8]**

| | Example 11 | Example 12 | Example 13 | Example 14 | Comparative Example 6 |
|---|---|---|---|---|---|
| Method of adding lubricant | Internal mixing | Internal mixing | Internal mixing | Internal mixing | Internal mixing |
| Ethenzamide | 10 | 10 | 10 | 10 | 10 |
| Carmellose | 10 | 10 | 10 | 10 | 10 |
| Crystalline cellulose | 31 | 31 | 31 | 31 | 31 |
| Anhydrous dibasic calcium phosphate | 47.6 | 47.6 | 47.6 | 47.6 | 47.6 |
| Acesulfame potassium | 1.4 | 1.4 | 1.4 | 1.4 | 1.4 |
| Magnesium stearate | 0.1 | 0.3 | 0.5 | 0.8 | 1 |
| Total amount (mg) | 100.1 | 100.3 | 100.5 | 100.8 | 101 |

### (Experimental result)

Experimental results are shown in Table 9. From this result, there was a tendency that as an addition amount of magnesium stearate was increased as 0.1 mg, 0.3 mg, 0.5 mg, 0.8 mg and 1 mg, a disintegration time and an intraoral disintegration time were delayed.

**[Table 9]**

| | Example 11 | Example 12 | Example 13 | Example 14 | Comparative Example 6 |
|---|---|---|---|---|---|
| Method of adding lubricant | Internal mixing | Internal mixing | Internal mixing | Internal mixing | Internal mixing |
| Hardness (N) | 63.3 | 61.3 | 59.8 | 61.0 | 65.0 |
| Disintegration time (sec) | 9 | 13 | 15 | 12 | 21 1 |
| Intraoral disintegration time (sec) | 9 | 10 | 10 | 14 | 24 |

### (Stability with time test of tablets in which an addition amount of a lubricant is changed)

Components of Table 8 were compressed at a compression pressure of 7 kN, the tablets were allowed to stand in an oven at 40°C and a relative humidity of 75% for 9 days, and hardness of a tablet and a disintegration time of a tablet were measured.

### (Experimental result)

Experimental results are shown in Table 10. From this result, when an addition amount of magnesium stearate which is a lubricant is 0.1 mg, 0.3 mg, 0.5 mg, or 0.8 mg, a tablet hardness is not lower than 30 N, a disintegration time is not longer than 30 seconds, and both of them attained a goal value. On the other hand, in the case of 1.0 mg, a tablet hardness was less than 30 N, and a disintegration time was not shorter than 30 seconds.

**[Table 10]**

| | Example 11 | Example 12 | Example 13 | Example 14 | Comparative Example 6 |
|---|---|---|---|---|---|
| Magnesium stearate (mg/tablet) | 0.1 | 0.3 | 0.5 | 0.8 | 1.0 |
| Hardness after 9 days (N) | 37.2 | 34.5 | 30.0 | 31.0 | 23.0 |
| Disintegration time after 9 days (sec) | 8 | 16 | 25 | 28 | 31 |

### (Study of kind of lubricant)

Formulation of Examples 15 to 18 is shown in Table 11. Hardness, a disintegration time and an intraoral disintegration time of tablets in which a kind of a lubricant was changed were measured. A method of producing tablets of Examples 15 to 18 is an external lubricating method as in Example 1, and the tablet was produced at a compression pressure of 6 kN.

**[Table 11]**

| | Example 15 | Example 16 | Example 17 | Example 18 |
|---|---|---|---|---|
| Ethenzamide | 10 | 10 | 10 | 10 |
| Carmellose | 10 | 10 | 10 | 10 |
| Crystalline cellulose | 31 | 31 | 31 | 31 |
| Anhydrous dibasic calcium phosphate | 47.6 | 47.6 | 47.6 | 47.6 |
| Acesulfame potassium | 1.4 | 1.4 | 1.4 | 1.4 |
| Sucrose fatty acid ester | 0.1 | - | - | - |
| Talc | - | 0.1 | - | - |
| Hydrated silicon dioxide | - | - | 0.1 | - |
| Magnesium stearate | - | - | - | 0.1 |
| Total amount (mg) | 100.1 | 100.1 | 100.1 | 100.1 |

### (Experimental result)

Experimental results are shown in Table 12. From this result, even when a kind of a lubricant was changed, hardness was high, and a disintegration time and an intraoral disintegration time were short.

**[Table 12]**

| | Example 15 | Example 16 | Example 17 | Example 18 |
|---|---|---|---|---|
| Hardness (N) | 52.0 | 51.2 | 52.8 | 50.3 |
| Disintegration time (sec) | 7 | 8 | 8 | 7 |
| Intraoral disintegration time (sec) | 9 | 8 | 9 | 10 |

### (Study of addition amount of carmellose)

Formulation of Examples 1, and 19 to 21 in which an addition amount of carmellose was changed to 1 mg (1 weight %), 5 mg (5 weight %), 10 mg (10 weight %), and 30 mg (30 weight %) is shown in Table 13. The production method in Examples 1, and 19 to 21 is an external lubricating method as in Example 1, and the tablet was produced at a compression pressure of 6 kN.

**[Table 13]**

| Component | Example 1 | Example 19 | Example 20 | Example 21 |
|---|---|---|---|---|
| Ethenzamide | 10 | 10 | 10 | 10 |
| Carmellose | 10 | 1 | 5 | 30 |
| Crystalline cellulose | 31 | 35 | 33 | 23 |
| Anhydrous dibasic calcium phosphate | 47.6 | 52.6 | 50.6 | 35.6 |
| Acesulfame potassium | 1.4 | 1.4 | 1.4 | 1.4 |
| Magnesium stearate | Minor amount (0.1) | Minor amount (0.1) | Minor amount (0.1) | Minor amount (0.1) |
| Total amount (mg) | 100 | 100 | 100 | 100 |

### (Experimental result)

Experimental results are shown in Table 14. From this result, in tablets in which 1 mg, 5 mg, 10 mg and 30 mg of carmellose is added, no problem is recognized in hardness, a disintegration time and an intraoral disintegration time, and there was no dry feeling.

**[Table 14]**

| | Example 1 | Example 19 | Example 20 | Example 21 |
|---|---|---|---|---|
| Hardness (N) | 52.0 | 51.5 | 48.8 | 38.5 |
| Disintegration time (sec) | 5 | 11 | 14 | 14 |
| Intraoral disintegration time (sec) | 6 | 20 | 10 | 10 |
| Dry feeling | ○ | ○ | ○ | ○ |

### (Study of addition amount of active ingredient)

Formulation of Examples 1, and 22 to 25 in which an addition amount of ethenzamide which is an active ingredient was changed to 10 mg (10 weight %), 20 mg (20 weight %), 30 mg(30 weight %), 40 mg (40 weight %) and 50 mg (50 weight %) is shown in Table 15. The production method in Examples 1, and 22 to 25 is an external lubricating method as in Example 1, and the tablet was produced at a compression pressure of 6 kN.

**[Table 15]**

| Component | Example 1 | Example 22 | Example 23 | Example 24 | Example 25 |
|---|---|---|---|---|---|
| Ethenzamide | 10 | 20 | 30 | 40 | 50 |
| Carmellose | 10 | 10 | 10 | 10 | 10 |
| Crystalline cellulose | 31 | 27 | 24 | 20 | 16 |
| Anhydrous dibasic calcium phosphate | 47.6 | 41.6 | 34.6 | 28.6 | 22.6 |
| Acesulfame potassium | 1.4 | 1.4 | 1.4 | 1.4 | 1.4 |
| Magnesium stearate | Minor amount (0.1) | Minor amount (0.1) | Minor amount (0.1) | Minor amount (0.1) | Minor amount (0.1) |
| Total amount (mg) | 100 | 100 | 100 | 100 | 100 |

### (Experimental result)

Experimental results are shown in Table 16. From this result, even when a blending amount of ethenzamide which is an active ingredient was changed, hardness of all tablets is high, and a disintegration time was short.

**[Table 16]**

| | Example 1 | Example 22 | Example 23 | Example 24 | Example 25 |
|---|---|---|---|---|---|
| Hardness (N) | 52.0 | 50.8 | 54.0 | 51.8 | 56.0 |
| Disintegration time (sec) | 5 | 6 | 9 | 9 | 12 |
| Intraoral disintegration time (sec) | 6 | 6 | 8 | 9 | 10 |

### (Process for producing powder particle containing active ingredient)

Formulation of the present powder particle is shown in a granule of Table 17. Anhydrous dibasic calcium phosphate and carmellose calcium were added to a 2-type high speed mixer (Fukae Powtec Co., Ltd.), a liquid in which a predetermined amount of isopropylantipyrine which is a drug was suspended in a 20 w/w% aqueous solution of hydroxypropylmethylcellulose 2910 was added separately, and the mixture was stirred and granulated. The granule was dried with a FL-MINI fluidized bed granulator (Freund Corporation). A ratio of blending a drug and a bitter taste masking base was 1:3 as expressed by a weight ratio (solid matter). As a drug, isopropylantipyrine (manufactured by KONGO CHEMICAL CO., LTD.) was used. As hydroxypropylmethylcellulose 2910 which is a bitter taste masking base, TC-5EW (manufactured by Shin-Etsu Chemical Co., Ltd.) was used. As an excipient, anhydrous dibasic calcium phosphate (manufactured by Kyowa Chemical Industry Co., Ltd.) was used. As a disintegrating agent, carmellose calcium (manufactured by Gotoku Chemical Company LTD.) was used. A viscosity of a 20 w/w% aqueous solution of hydroxypropylmethylcellulose 2910 at 20°C is 50 to 14000 mPa·s.

### (Process for producing tablet)

The aforementioned powder particle, a disintegrating agent, crystalline cellulose, anhydrous dibasic calcium phosphate, and a sweetener were weighed according to a blending amount described in Table 17, and mixed with a V-type mixer (manufactured by Dalton Co., Ltd) to prepare a powder for tablet. Then, using an experimental small compressing machine rotary compressing machine (manufactured by KIKUSUI SEISAKUSHO LTD.) equipped with an external lubricating apparatus ELSP1-type III (manufactured by KIKUSUI SEISAKUSHO LTD.), tablets each weighing 100 mg were prepared under the condition that not more than 0.1 mg of magnesium stearate is adhered per tablet. Thereupon, a mallet having a round shape and a diameter of 6.5 mm was used, and a compression pressure was 5 to 7 kN.

### (Study of formulation using powder/particle in which active ingredient is coated)

### (Study of disintegrating agent)

Since when a powder/particle in which an active ingredient is coated is used to perform compression, it is thought that hardness of a tablet is reduced, and a disintegration time and an intraoral disintegration time are delayed, a disintegrating agent was studied again.

### (Examples 26, Comparative Examples 8 to 12)

In Example 26, and Comparative Examples 8 to 12, using formulation of Table 17 as fundamental formulation, powders for tablet in which a kind of a disintegrating agent was changed were compressed, and hardness, a disintegration time, an intraoral disintegration time, and dry feeling in an oral cavity of the tablets were assessed.

**[Table 17]**

| | Component | Blending amount |
|---|---|---|
| Granule | Isopropylantipyrine | 1 |
| | Hydroxypropylmethylcellulose 2910 | 3 |
| | Anhydrous dibasic calcium phosphate | 10 |
| | Carmellose calcium | 10 |
| Tablet component | Disintegrating agent | 10 |
| | Crystalline cellulose | 26 |
| | Anhydrous dibasic calcium phosphate | 38.5 |
| | Acesulfame potassium | 1.4 |
| | Magnesium stearate | Minor amount (0.1) |
| Total amount (mg) | | 100 |

### (Experimental result)

Experimental results are shown in Table 18. From this result, when carmellose is used as in Table 2, hardness of the tablet was high, a disintegration time and an intraoral disintegration time were short, and there was no dry feeling in an oral cavity. In the case of other disintegrating agents, a disintegration time in an oral cavity was long, and there was dry feeling.

**[Table 18]**

| | Example 26 | Comparative Example 8 | Comparative Example 9 | Comparative Example 10 | Comparative Example 11 | Comparative Example 12 |
|---|---|---|---|---|---|---|
| Disintegrating agent name | Carmellose | Carmellose calcium | Croscarmellose sodium | Low-substituted hydroxyporopylc ellulose | Carboxymethyl starch sodium | Crospovidone |
| Hardness (N) | 39.0 | 35.8 | 37.0 | 49.3 | 42.0 | 53.3 3 |
| Disintegration time (sec) | 12 | 20 | 38 | 18 | 29 | 13 |
| Intraoral disintegration time (sec) | 18 | 50 | 55 | 47 | 51 | 35 |
| Dry feeling | O | Δ | × | Δ | Δ | × |

### (Study of addition amount of powder particle in which active ingredient is coated)

### (Process for producing tablet)

Formulation of the tablet of Example 27 is shown in Table 19. Formulation is the same as that of Example 26 except that an addition amount of isopropylantipyrine was 2 mg, and a mallet having a diameter of 7.00 mm was used.

**[Table 19]**

| | | Example 26 | Example 27 |
|---|---|---|---|
| Granule | Isopropylantipyrine | 1 | 2 |
| | Hydroxypropylmethylcellulose 2910 | 3 | 6 |
| | Anhydrous dibasic calcium phosphate | 10 | 20 |
| | Carmellose calcium | 10 | 20 |
| Tablet component | Carmellose | 10 | 13 |
| | Crystalline cellulose | 26 | 27 |
| | Anhydrous dibasic calcium phosphate | 38.6 | 40.2 |
| | Acesulfame potassium | 1.4 | 1.8 |
| | Magnesium stearate | Minor amount (0.1) | Minor amount (0.13) |
| Total amount (mg) | | 100 | 130 |

### (Experimental result)

Experimental results are shown in Table 20. From this result, even when a blending amount of an active ingredient is increased, hardness of the tablet was high, and a disintegration time and an intraoral disintegration time were short as in a 1mg tablet of Example 26.

**[Table 20]**

| | Example 26 | Example 27 |
|---|---|---|
| Hardness (N) | 39.0 | 41.0 |
| Disintegration time (sec) | 12 | 15 |
| Intraoral disintegration time (sec) | 18 | 25 |

### (Study of addition of perfume)

### (Process for producing tablet)

Formulation of the tablet of Examples 28 and 29 is shown in Table 21. Formulation is the same as that of Examples 26 and 27 except that a mint oil which is a perfume, and hydrated silicon dioxide were blended.

**[Table 21]**

| | | Example 28 | Example 29 |
|---|---|---|---|
| Granule | Isopropylantipyrine | 1 | 2 |
| | Hydroxypropylmethylcellulose 2910 | 3 | 6 |
| | Anhydrous dibasic calcium phosphate | 10 | 20 |
| | Carmellose calcium | 10 | 20 |
| Tablet component | Carmellose | 10 | 13 |
| | Crystalline cellulose | 26 | 27 |
| | Anhydrous dibasic calcium phosphate | 38.4 | 39.94 |
| | Acesulfame potassium | 1.4 | 1.8 |
| | Magnesium stearate | Minor amount (0.1) | Minor amount (0.13) |
| | Mint oil | Minor amount (0.1) | Minor amount (0.13) |
| | Hydrated silicon dioxide | 0.2 | 0.26 |
| Total amount (mg) | | 100 | 130 |

### (Experimental result)

Experimental results are shown in Table 22. From this result, even when a perfume was blended, hardness of the tablet was high, and a disintegration time and an intraoral disintegration time were short.

**[Table 22]**

| | Example 28 | Example 29 |
|---|---|---|
| Hardness (N) | 40.0 | 39.2 |
| Disintegration time (sec) | 13 | 14 |
| Intraoral disintegration time (sec) | 19 | 24 |

### (Study of amount of drug and additive of powder particle in which active ingredient is coated)

### (Process for producing tablet)

Formulation of the tablet of Examples 31, 32 and 33 is shown in Table 23. Formulation is the same as that of Examples 28 and 29 except that an addition amount of a drug and an additive was changed.

**[Table 23]**

| | | Example 31 | Example 32 | Example 33 |
|---|---|---|---|---|
| Granule | Isopropylantipyrine | 0.5 | 3 | 3 |
| | Hydroxypropylmethylcellulose 2910 | 1.5 | 9 | 9 |
| | Anhydrous dibasic calcium phosphate | 5 | 30 | 30 |
| | Carmellose calcium | 5 | 30 | 30 |
| Tablet component | Carmellose | 5 | 19.5 | 30 |
| | Crystalline cellulose | 13 | 40.5 | 78 |
| | Anhydrous dibasic calcium phosphate | 19.2 | 59.91 | 115.2 |
| | Acesulfame potassium | 0.7 | 2.7 | 4.2 |
| | Magnesium stearate | Minor amount (0.05) | Minor amount (0.19) | Minor amount (0.3) |
| | Mint oil | Minor amount (0.05) | Minor amount (0.19) | Minor amount (0.3) |
| | Hydrated silicon dioxide | 0.1 | 0.39 | 0.6 |
| Total amount (mg) | | 50 | 195 | 300 |

A mallet diameter and a compression pressure of a compressing machine in Examples 31, 32 and 33 are as described in Table 24.

**[Table 24]**

| | Example 31 | Example 32 | Example 33 |
|---|---|---|---|
| Mallet diameter (mm) | 5.0 | 7.5 | 8.5 |
| Compression pressure (kN) | 7.0 | 7.5 | 5.5 |

### (Experimental result)

Experimental results are shown in Table 25. From this result, as in Examples 28 and 29, hardness of any tablet was not lower than 30 N, and a disintegration time was within 30 seconds.

**[Table 25]**

| | Example 31 | Example 32 | Example 33 |
|---|---|---|---|
| Hardness (N) | 42 | 51 | 60 |
| Disintegration time (sec) | 13 | 22 | 21 |

### (Study of kind and addition amount of drug)

### (Process for producing tablet by method of directly compressing powder)

Formulation of a tablet is shown in Tables 26, 27 and 28. As a drug, acetaminophen, cefcapene pivoxil hydrochloride and rilmazafone hydrochloride were used. As a process for producing a tablet, a drug, carmellose, crystalline cellulose, anhydrous dibasic calcium phosphate, acesulfame potassium, and magnesium stearate were weighed according to a blending amount described in Tables 26, 27 and 28, and mixed in a polyethylene bag to prepare a powder for tablet. Then, using an experimental small compressing machine VELA5 (manufactured by KIKUSUI SEISAKUSHO LTD.) equipped with an external lubricating apparatus ELSP1-type III (manufactured by KIKUSUI SEISAKUSHO LTD.), tablets each weighing 100 mg were produced under the condition that not more than 0.1 mg of magnesium stearate was adhered per tablet. Thereupon, a mallet having a round shape and a diameter of 6.5 mm was used, and the powder was compressed at a compression pressure of 6 kN.

**[Table 26]**

| Component | Example 34 | Example 35 | Example 36 |
|---|---|---|---|
| Acetaminophen | 10 | 30 | 50 |
| Carmellose | 10 | 10 | 10 |
| Crystalline cellulose | 31 | 24 | 16 |
| Anhydrous dibasic calcium phosphate | 47.6 | 34.6 | 22.6 |
| Acesulfame potassium | 1.4 | 1.4 | 1.4 |
| Magnesium stearate | Minor amount (0.1) | Minor amount (0.1) | Minor amount (0.1) |
| Total amount (mg) | 100 | 100 | 100 |

**[Table 27]**

| Component | Example 37 | Example 38 | Example 39 |
|---|---|---|---|
| Cefcapene pivoxil hydrochloride | 10 | 30 | 50 |
| Carmellose | 10 | 10 | 10 |
| Crystalline cellulose | 31 | 24 | 16 |
| Anhydrous dibasic calcium phosphate | 47.6 | 34.6 | 22.6 |
| Acesulfame potassium | 1.4 | 1.4 | 1.4 |
| Magnesium stearate | Minor amount (0.1) | Minor amount (0.1) | Minor amount (0.1) |
| Total amount (mg) | 100 | 100 | 100 |

**[Table 28]**

| Component | Example 40 | Example 41 | Example 42 |
|---|---|---|---|
| Rilmazafone hydrochloride | 10 | 30 | 50 |
| Carmellose | 10 | 10 | 10 |
| Crystalline cellulose | 31 | 24 | 16 |
| Anhydrous dibasic calcium phosphate | 47.6 | 34.6 | 22.6 |
| Acesulfame potassium | 1.4 | 1.4 | 1.4 |
| Magnesium stearate | Minor amount (0.1) | Minor amount (0.1) | Minor amount (0.1) |
| Total amount (mg) | 100 | 100 | 100 |

### (Experimental result)

Experimental results of each drug are shown in Tables 29, 30 and 31. From this result, there was a tendency that as a drug amount was increased, hardness of a tablet was reduced, and a disintegration time becomes longer, but all tablets had hardness of not lower than 30 N, and a disintegration time within 30 seconds.

**[Table 29]**

| Acetaminophen | | | |
|---|---|---|---|
| | Example 34 | Example 35 | Example 36 |
| Hardness (N) | 38 | 36 | 34 |
| Disintegration time (sec) | 12 | 17 | 25 |

**[Table 30]**

| Cefcapene pivoxil hydrochloride | | | |
|---|---|---|---|
| | Example 37 | Example 38 | Example 39 |
| Hardness (N) | 50 | 47 | 43 |
| Disintegration time (sec) | 12 | 13 | 18 |

**[Table 31]**

| Rilmazafone hydrochloride | | | |
|---|---|---|---|
| | Example 40 | Example 41 | Example 42 |
| Hardness (N) | 38 | 37 | 32 |
| Disintegration time (sec) | 7 | 13 | 20 |

### Industrial Applicability

The tablet of the present invention is easy to produce, has both strengths at production and storage, and is excellent in storage and stability for a long term. In addition, since the tablet is rapidly disintegrated in an oral cavity, the tablet can be used in treating and preventing a variety of diseases like the previous oral agent containing the same drug, as a preparation which is easily ingested by an elderly or an infant, or as a safe preparation for a general person.

## Claims

1. A tablet **characterized in that** an active ingredient, crystalline cellulose, inorganic excipient, carmellose and a lubricant at not more than 0.8% by weight per tablet are contained.

2. The tablet according to claim 1, wherein the inorganic excipient is anhydrous dibasic calcium phosphate.

3. The tablet according to claim 1 or 2, wherein a lubricant at not more than 0.5% by weight per tablet is blended.

4. The tablet according to claim 3, wherein a lubricant at not more than 0.1% by weight per tablet is blended.

5. The tablet according to any one of claims 1 to 4, wherein an average particle diameter of crystalline cellulose is 10 to 150 µm.

6. The tablet according to any one of claims 2 to 5, wherein a bulk density of anhydrous dibasic calcium phosphate is 0.3 to 1.0 g/mL.

7. The tablet according to any one of claims 1 to 6, wherein carmellose is blended at 1 to 30% by weight per tablet.

8. The tablet according to any one of claims 1 to 7, wherein the lubricant is a satiric acid metal salt.

9. The tablet according to claim 8, wherein the stearic acid metal salt is magnesium stearate.

10. The tablet according to any one of claims 1 to 9, wherein a sweetener is blended.

11. The tablet according to claim 10, wherein the sweetener has a sweetness degree which is 50-fold more, letting a sweetness of white sugar to be 1.

12. The tablet according to claim 11, wherein the sweetener is acesulfame potassium or sucralose.

13. The tablet according to any one of claims 10 to 12, wherein a blending amount of the sweetener is not more than 10% by weight per tablet.

14. The tablet according to any one of claims 1 to 13, wherein a method of adding a lubricant is an external lubricating method.

15. The tablet according to claim 14, wherein an active ingredient, crystalline cellulose, anhydrous dibasic calcium phosphate, carmellose, and magnesium stearate at not more than 0.1% by weight per tablet are contained.

16. The tablet according to any one of claims 1 to 15, which is an orally disintegrating tablet.
